# EUROPÄISCHE PATENTSCHRIFT

(11) **EP 0 467 045 B1**
(45) Veröffentlichungstag und Bekanntmachung des Hinweises auf die Patenterteilung: **04.03.1998**
(21) Anmeldenummer: 91108430.9
(22) Anmeldetag: 24.05.1991
(51) Int. Cl.: C07D 265/30, C07D 265/36, C07D 295/14, A01N 47/38

(54) **Morpholinoharnstoff-Derivate**
Morpholino urea derivatives
Dérivés de morpholino-urées

(30) Priorität: 06.06.1990 DE 4018070
(43) Veröffentlichungstag der Anmeldung: 22.01.1992
(73) Patentinhaber: BAYER AG, 51368 Leverkusen (DE)
(72) Erfinder: Stroech, Klaus, Dr., W-5650 Solingen 19 (DE); Krüger, Bernd-Wieland, Dr., W-5060 Bergisch Gladbach (DE); Hoever, Peter, Dr., W-5000 Köln 80 (DE); Nentwig, Günther, Dr., W-5000 Köln 80 (DE)

(56) Entgegenhaltungen:
- EP-A- 0 085 286
- EP-A- 0 126 934
- EP-A- 0 196 570
- DE-A- 2 206 366
- DE-A- 3 539 994
- FR-A- 2 050 522
- US-A- 3 161 676
- US-A- 4 025 652
- US-A- 4 107 436
- US-A- 4 356 180
- US-A- 4 400 512
- LIEBIGS ANN. CHEM. 1984, Seiten 108-126, Verlag Chemie GmbH, Weinheim, DE; W. KANTLEHNER et al.: "Herstellung von 1,1,2,3,3-pentasubstituierten und 1,1,2,2,3,3-hexasubstituierten Guanidiniumsalzen sowie von 1,1,2,3,3-Pentaalkylguanidinen"
- CHEMICAL ABSTRACTS Band 67, Nr. 11, 11. September 1967, Zusammenfassung Nr. 54097p, Columbus, Ohio, US: V.I. GUNAR et al: "Synthesis of 1,3-oxazine derivatives on the basis of diketene" & Khim. Geterotrikl. Soedin. 1967, Band 1, Seiten 48-50
- CHEMICAL ABSTRACTS Band 102, Nr. 3, 21. Januar 1985, Zusammenfassung Nr. 24560h, Columbus, Ohio, US; P. MELLONI et al.: "Potential antidepressant agents. Alpha-Aryloxy-benzyl derivatives of ethanolamine and morpholine" && J. Med. Chem.--Chim. Ther. 1984, Band 19, Nr. 3,Seiten 235-242
- CHEMICAL ABSTRACTS Band 107, Nr. 9, 31. August 1987, Zusammenfassung Nr. 70331k, Columbus, Ohio, US; N.D. DORIA et al.: "Filariasis: Endemic disease in search of effective drugs" & Quim. Nova 1986, Band 9, Nr. 4, Seiten 273-275 *
- CHEM. PHARM. BULL. Band 37, Nr. 8, 1989, Seiten 2026-2029, JP; T. KINOSHITA et al.: "Ring Cleavage Reaction of 1,3-Oxazine-2,4(3H)-dione Derivatives with Amines"
- CHEM. BER. Band 106, 1973, Seiten 2315-2323, DE; H. GROSS et al.: "Ueber die Raktion 1,3-disubstituierter 2,2-Dichlor-4,5-imidazolidindione mit sek. Aminen"
- CHEM SOC REV, 1979, 563-80
- EUR.J.MED.CHEM - CHIM THER., 1986, 21 (5), 439-44
- ALDRICH-KATALOG, 1990, SEITE 396

## Beschreibung

Die Erfindung betrifft neue Morpholinoharnstoff-Derivate, mehrere Verfahren zu ihrer Herstellung und ihre Verwendung als insekten- und milbenabwehrende Mittel.

Mittel, die Insekten und Milben abwehren (Repellents), haben die Aufgabe, schädliche oder lästige Gliederfüßler von Berührung, sowie vom Stechen und Saugen oder Beißen an für sie anlockende Oberflächen, etwa der Haut von Tieren und Menschen abzuhalten, wenn diese zuvor mit solchen Mitteln behandelt wurden.

Als Repellents wurden bereits zahlreiche Wirkstoffe vorgeschlagen. (Vergl. z.B. K.H. Büchel in Chemie der Pflanzenschutz- und Schädlingsbekämpfungsmittel; Herausgeber: R. Wegler, Bd. 1, Springer Verlag Berlin, Heidelberg, New York, 1970, S. 487 ff).

Besonders bekannt und seit längerer Zeit in Gebrauch sind 3-Methyl-benzoesäurediethylamid (DEET), Dimethylphthalat phthalat und 2-Ethyl-hexandiol-1,3, von denen vor allem das DEET in der Praxis eine erhebliche Bedeutung erlangt hat (siehe z.B. R.K. Kocher, R.S. Dixit, C.I. Somaya; Indian J. Med. Res. 62, 1 (1974)):

Weiterhin sind Harnstoff-Derivate und Carboxamide bekannt, die eine insektenabweisende Wirkung besitzen (siehe EP-A 22 653; DE-A 27 56 360; US-Patent Nr. 3 624 204 sowie US-Patent Nr. 4 356 180).

Harnstoff-Derivate und Carboxamide sind auch aus FR-A 2 050 522, DE-A-3 539 994, DE-A- 2 206 366, Chem. Ber. 106 (1973), S. 2315 - 2323, C. A. 107 (1987), 70331 k, EP-A- 0 085 286, US-A- 4 107 436, US-A- 4 025 652, US-A-3 161 676, C. A. 67 (1967), 54097 p, C. A. 102 (1985), 24560 h, Chem. Pharm. Bull. 37 (1989), S. 2026 - 2020 und US-A- 4 356 180 bekannt, wobei eine insekten- und milbenabwehrende Wirkung nicht beschrieben wurde.

Ein erheblicher Nachteil der bekannten Repellents ist ihre zum Teil relativ kurze (nur wenige Stunden) anhaltende Dauerwirkung.

Es wurden nun neue Morpholinoharnstoff-Derivate der Formel (I) 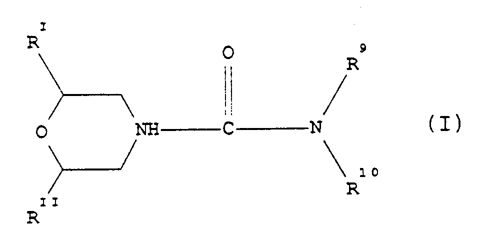 gefunden, in welcher
- R^{I} und R^{II}: gleich oder verschieden sind und für Wasserstoff, C₁-C₆-Alkyl, C₂-C₆-Alkenyl, C₃-C₆-Cylcoalkyl oder Phenyl stehen und
- R⁹: für gegebenenfalls durch C₁-C₄-Alkoxy oder Halogen-substituiertes C₁-C₆-Alkyl, Alkenyl mit bis zu 6 C-Atomen, C₃-C₆-Cycloalkyl, Benzyl oder Cyclohexylmethyl steht,
- R¹⁰: für gegebenenfalls durch C₁-C₄-Alkoxy oder Halogen substituiertes C₁-C₆-Alkyl, C₃-C₆-Cycloalkyl oder Benzyl steht,
oder
- R⁹ und R¹⁰: zusammen für einen gegebenenfalls durch Sauerstoff unterbrochenen und gegebenenfalls durch C₁-C₄-Alkyl substituierten Tetramethylen-, Pentamethylen-, Hexamethylen- oder Heptamethylenrest stehen,
wobei solche Verbindungen der Formel (I) ausgenommen sind, in welchen zugleich
a)
   R^{I} und R^{II} für Wasserstoff und
   R⁹ und R¹⁰ für Methyl oder Ethyl stehen und
b)
   R^{I} und R^{II} für Wasserstoff stehen und
   R⁹ und R¹⁰ einen Morpholinring bilden.

Weiterhin wurde gefunden, daß man die Morpholinoharnstoff-Derivate der Formel (I) erhält,
indem man entweder
a) Morpholine der Formel (II) 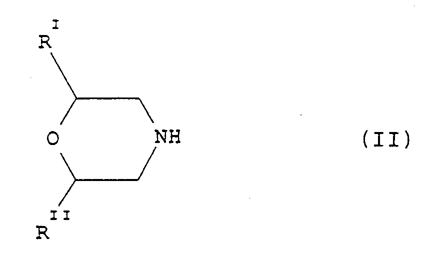 worin
   R^{I} und R^{II} die oben angegebene Bedeutung haben,
      mit Carbamidsäurederivaten der Formel (III) 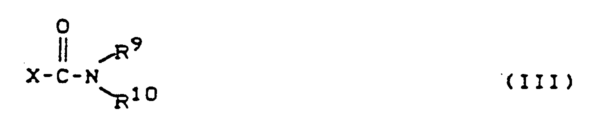 in welcher
   R⁹ und R¹⁰ die oben angegebene Bedeutung besitzen und
   X für eine Abgangsgruppe steht,
      in Gegenwart einer Base und in Gegenwart eines Lösungsmittels umsetzt,
   oder indem man
b) zunächst Morpholine der Formel (II) 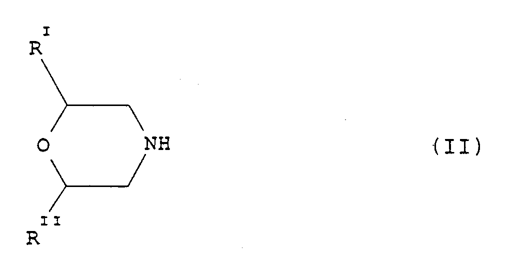 worin
   R^{I} und R^{II} die oben angegebene Bedeutung haben,
      mit Isocyanaten der Formel (IV)

      O=C=N-R⁹ (IV)

      worin
   R⁹ die oben angegebene Bedeutung besitzt,
      in einem Lösungsmittel umsetzt und das erhaltene Produkt der Formel (V) 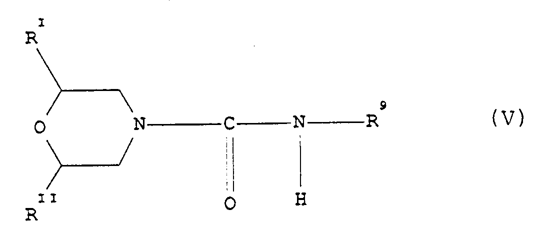 mit einem Alkylierungsmittel der Formel (VI)

      R¹⁰-X (VI)

      worin
   R¹⁰ die oben angegebene Bedeutung besitzt und
   X für eine Abgangsgruppe steht,
   in einem Lösungsmittel in Gegenwart einer Base umsetzt,
   oder indem man
c) 4-Halogencarbonylmorpholin-Derivate der Formel (VII) 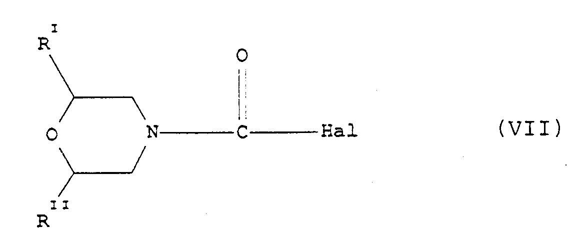 worin
   R^{I} und R^{II} die obengenannte Bedeutung haben
      und Hal für Halogen, insbesondere für Chlor, steht, mit
      sekundären Aminen der Formel (VIII) 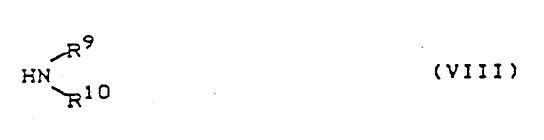 worin
   R⁹ und R¹⁰ die obengenannte Bedeutung haben,
      gegebenenfalls in Gegenwart von Basen und gegebenenfalls in Gegenwart von Lösungsmitteln umsetzt.

Weiterhin wurde gefunden, daß sich die neuen Morpholinoharnstoff-Derivate der Formel (I), einschließlich der Verbindungen der Formel (I), in welchen zugleich
a)
   R^{I} und R^{II} für Wasserstoff und
   R⁹ und R¹⁰ für Methyl oder Ethyl stehen und
b)
   R^{I} und R^{II} für Wasserstoff stehen und
   R⁹ und R¹⁰ einen Morpholinring bilden,
durch eine sehr stark ausgeprägte insekten- und milbenabwehrende Wirkung auszeichnen.

Die Wirkung als Repellent ist erheblich besser als die der aus dem Stand der Technik bekannten Repellents. Die erfindungsgemäßen Wirkstoffe stellen somit eine wertvolle Bereicherung der Technik dar.

Die in der Formel (I) angegebenen Reste haben vorzugsweise die folgende Bedeutung:

Gegebenenfalls substituiertes Alkyl in den Resten R^{I}, R^{II}, R⁹ und R¹⁰ ist geradkettig oder verzweigt und enthält 1 bis 6 Kohlenstoffatome. Beispielhaft seien Methyl, Ethyl, n- und i-Propyl, n-, i- und t-Butyl, n-Pentyl und n-Hexyl genannt.

Als gegebenenfalls substituiertes Alkenyl in den Resten R^{I}, R^{II} und R⁹ steht geradkettiges oder verzweigtes Alkenyl mit bis zu 6 Kohlenstoffatomen. Beispielhaft seien gegebenenfalls substituiertes Ethenyl, Propenyl-(1), Propenyl-(2), Butenyl-(1), Butenyl-(2) und Butenyl-(3) genannt.

Als gegebenenfalls substituiertes Cycloalkyl in den Resten R^{I}, R^{II} und R¹⁰ steht Cycloalkyl mit 3 bis 6, insbesondere 3, 5 oder 6 Kohlenstoffatomen. Beispielhaft seien gegebenenfalls substituiertes Cyclopropyl, Cyclobutyl, Cyclopentyl und Cyclohexyl genannt.

Halogen hat überall, wo nicht anders beschrieben, die Bedeutung Fluor, Chlor, Brom, Iod, insbesondere Fluor oder Chlor.

Die Reste Alkyl in R⁹ und R¹⁰ sowie Alkenyl und Cycloalkyl in R⁹ können einen oder mehrere, vorzugsweise 1 bis 3, insbesondere 1 oder 2 gleiche oder verschiedene Substituenten tragen. Als Substituenten seien beispielhaft aufgeführt : Alkoxy mit 1 bis 4, insbesondere 1 oder 2 Kohlenstoffatomen, wie Methoxy, Ethoxy, n- und i-Propyloxy und n-, i- und t-Butyloxy und Halogen, vorzugsweise Fluor, Chlor, Brom und Jod, insbesondere Chlor und Brom.

Falls R⁹ und R¹⁰ zusammen für einen gegebenenfalls durch Heteroatome unterbrochenen Polyalkylenring stehen, so stehen sie zusammen mit dem eingeschlossenen Stickstoffatom vorzugweise für folgende 5-bis 7-gliedrige, vorzugsweise 5-oder 6-gliedrige Ringsysteme: Pyrrolidin, Hexamethylonimin, Morpholin.

Bevorzugt sind Verbindungen der Formel (I) in welcher
- R^{I} und R^{II}: gleich oder verschieden sind und für Wasserstoff, Methyl, oder Ethyl stehen,
- R⁹: für C₁-C₆-Alkyl, Alkenyl mit bis zu 6 C-Atomen oder C₃-C₆-Cycloalkyl steht und
- R¹⁰: für C₁-C₆-Alkyl steht.

Außer den in den Herstellungsbeispielen aufgeführten seien noch die folgenden Morpholinharnstoff-Derivate genannt.

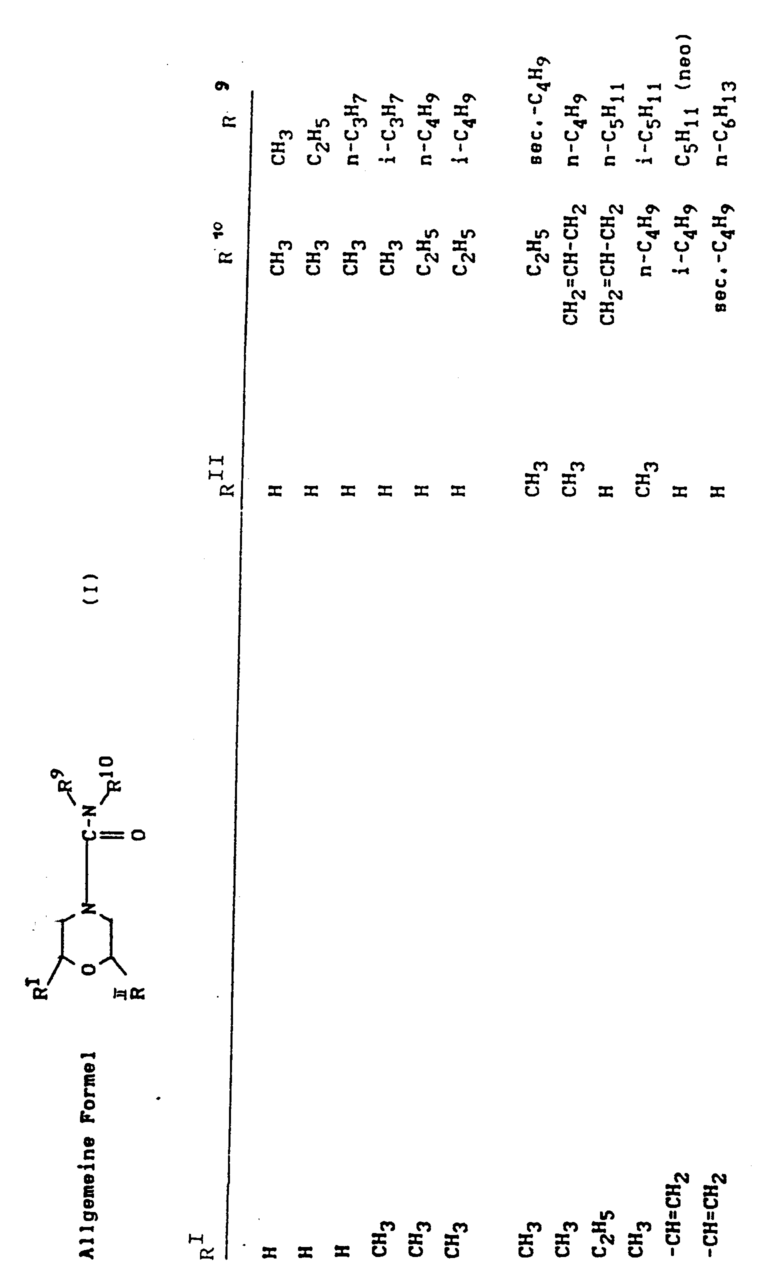

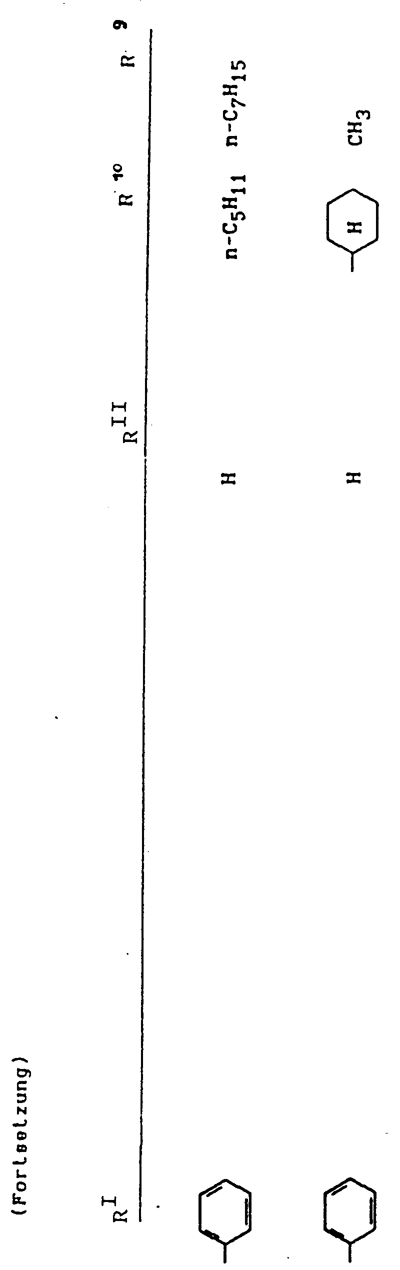

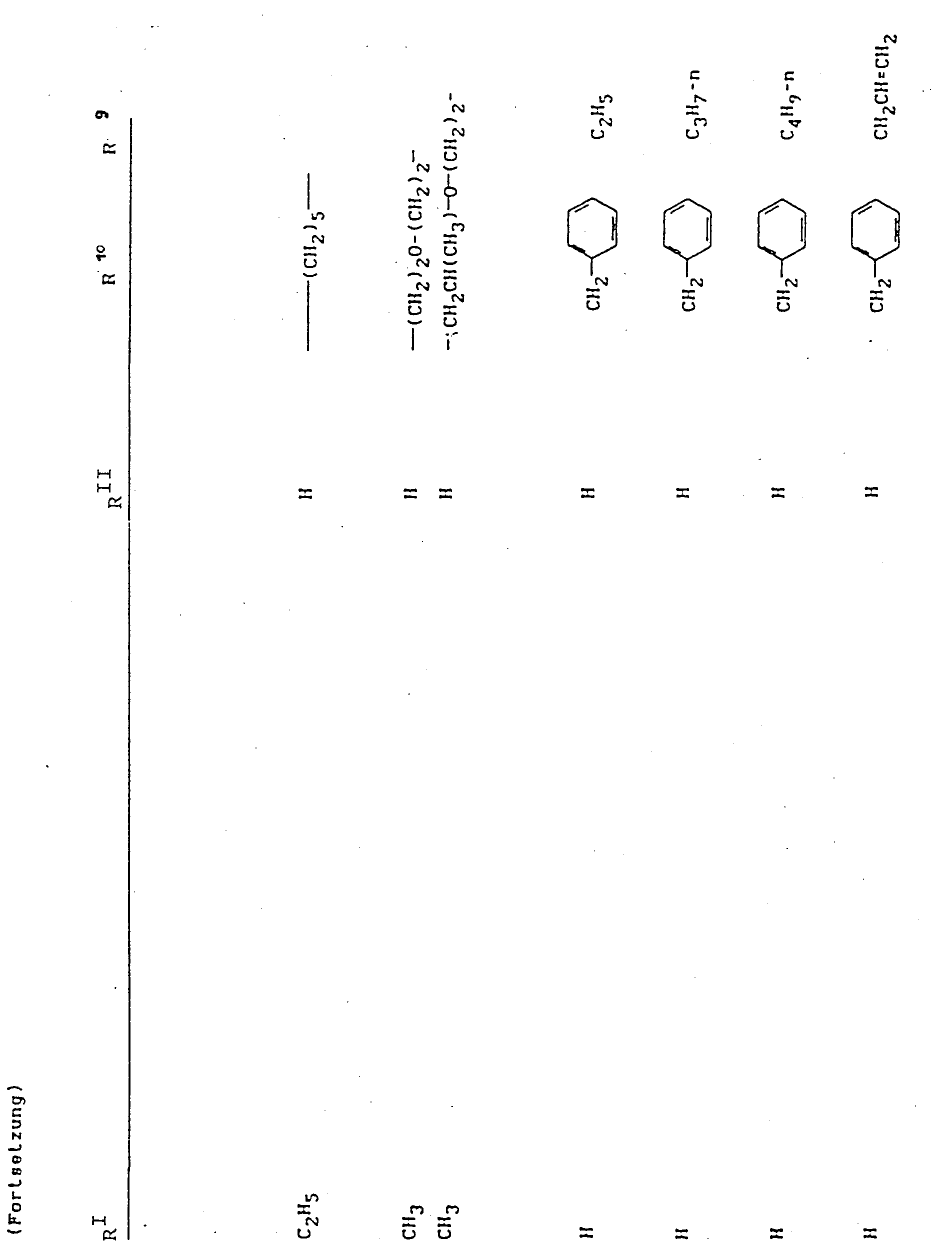

Die Umsetzung von Morpholinen der Formel (II) mit Carbamidsäurederivaten der Formel (III) wird bei der Herstellungsvariante a) in Gegenwart einer Base (vorzugsweise tertiäre organisches Amin wie z.B. Triethylethylamin, stickstoffhaltiger Aromat, wie z.B. Pyridin oder einer anorgan. Base wie NaOH oder KOH oder in Gegenwart eines Überschusses des Morpholinderivats (II)) gegebenenfalls in Gegenwart eines Katalysators (wie z.B. 4-Dimethylamino-pyridin oder 1,3-Diazabicyclo(2,2,2)-octan (DABCO) und in Gegenwart eines Lösungsmittels durchgeführt.

Als Lösungsmittel kommen z.B. inerte organische Lösungsmittel wie Ether, Benzol, Toluol in Frage. Gegebenenfalls kann auch Wasser als Lösungsmittel oder das Morpholinderivat (II) im Überschuß als Lösungsmittel eingesetzt werden.

Die Reaktionstemperatur beträgt -20°C bis 100°C und vorzugsweise 0 bis 60°C.

Jeder der Reaktionspartner (II) und (III) kann im Überschuß eingesetzt werden, vorzugsweise arbeitet man jedoch im Molverhältnis (II):(III) = 1:1.

Bei der Reaktionsvariante b) werden zunächst Morpholine der Formel (II) mit Isocyanaten der Formel (IV) in einem Lösungsmittel, vorzugsweise in einem inerten organischen Lösungsmittel wie z.B. Ether, Benzol, Toluol bei Temperaturen von 0 bis 150°C bevorzugt bei 20 bis 80°C umgesetzt. Gegebenenfalls wird ein Katalysator zugesetzt. Als Beispiel sei 1,8-Diazabicyclo(5,4,0)undec-7-en (DBU) genannt.

Das Molverhältnis Morpholinderivat (II) : Isocyanat (IV) beträgt vorzugsweise (1-1,2) : 1.

Die anschließende Umsetzung der Reaktionsprodukte der Formel (V) mit einem Alkylierungsmittel der Formel R¹⁰-X (VI), worin die Abgangsgruppe X bevorzugt für Chlor, Brom oder Jod steht, wird vorzugsweise in einem inerten organischen Lösungsmittel durchgeführt.

Beispielhaft seien Dimethylformamid, Dimethylsulfoxid, Ether, Acetonitril, Tetrahydrofuran genannt.

Als Basen werden übliche anorganische oder organische Basen zugesetzt. Beispielhaft seien genannt: NaOH, KOH, NaH, Butyllithium, Pyridin.

Die Reaktionstemperatur beträgt vorzugsweise -20°C bis 100°C, insbesondere 20 bis 80°C.

Das Alkylierungsmittel (VI) kann in äquimolarer Menge bzw. in bis zu 5-molarem Überschuß zur Verbindung (V) hinzugegeben werden.

Bei der Reaktionsvariante c) werden 4-Halogencarbonyl-Morpholin-Derivate der Formel (VII) mit sekundären Aminen der Formel (VIII) umgesetzt. Als Lösungsmittel, Basen und Katalysatoren können die bei der obigen Reaktionsvariante a) beschriebenen Lösungsmittel, Basen und Katalysatoren verwendet werden. Die Reaktionsbedingungen entsprechen den bei der Reaktionsvariante a) beschriebenen.

Die Aufarbeitung der gemäß Verfahrensvariante a), b) oder c) hergestellten Endprodukte der Formel (I) geschieht in an sich bekannter Weise, z.B. durch Extraktion im organischen Lösungsmitteln und anschließende Destillation bzw. Umkristallisation.

Die erfindungsgemäßen Mittel, die mindestens ein Derivat der Formel I enthalten, können auch weitere Insektenabwehrmittel enthalten. Hier kommen alle praktisch üblichen Repellentien in Frage (vgl. z.B. K.H. Büchel in Chemie der Pflanzenschutz- und Schädlingsbekämpfungsmittel; Herausg.: R. Wegler, Bd. 1, Springer Verlag Berlin, Heidelberg, New York, 1970, S. 487 ff).

Im Falle der Repellentkombinationen werden bevorzugt die Morpholinoharnstoff-Derivate der allgemeinen Formel I zusammen mit repellenten Carbonsäureamiden, 1,3-Alkandiolen und Carbonsäureestern verwendet. Im einzelnen seien genannt: 3-Methyl-benzoesäurediethylamid (DEET), 2-Ethyl-hexandiol-1,3 (Rutgers 612)® und Phthalsäuredimethylester.

Die insekten- und milbenabwehrende Wirkung der Verbindungen der allgemeinen Formel (I) hält lange an.

Sie können daher mit gutem Erfolg zur Abwehr von schädlichen oder lästigen, saugenden und beißenden Insekten und Milben verwendet werden.

Zu den saugenden Insekten gehören im wesentlichen die Stechmücken (z.B. Aedes-, Culex und Anopheles-Arten), Schmetterlingsmücken (Phlebotomen), Gnitzen (Culicoides-Arten), Kriebelmücken (Simulium-Arten), Stechfliegen (z.B. Stomoxys calcitrans), Tsetse-Fliegen (Glossina-Arten), Bremsen (Tabanus-, Haematopota- und Chrysops-Arten), Stubenfliegen (z.B. Musca domestica und Fannia canicularis), Fleischfliegen (z.B. Sarcophaga carnaria), Myiasis erzeugende Fliegen (z.B. Lucilia cuprina, Chrysomyia chloropyga, Hypoderma bovis, Hypoderma lineatum, Dermatobia hominis, Oestrus ovis, Gasterophilus intestinalis, Cochliomyia hominivorax), Wanzen (z.B. Cimex lectularius, Rhodnius prolixus, Triatoma infestans), Läuse (z.B. Pediculus humanus, Haematopinus suis, Damalina ovis), Lausfliegen (z.B. Melophagus ovinus), Flöhe (z.B. Pulex irritans, Ctenocephalides canis, Xenopsylla cheopis) und Sandflöhe (z.B. Dermatophilus penetrans).

Zu den beißenden Insekten gehören im wesentlichen Schaben (z.B. Blattella germanica, Periplaneta americana, Blatta orientalis, Supella supellectilium), Käfer (z.B. Sitophilus granarius, Tenebrio molitor, Dermestes lardarius, Stegobium paniceum, Anobium puntactum, Hylotrupes bajulus), Termiten (z.B. Reticulitermes lucifugus) und Ameisen (z.B. Lasius niger).

Zu den Milben gehören Zecken (z.B. Ornithodorus moubata, Ixodes ricinus, Boophilus microplus, Amblyomma hebreum) und Milben in engerem Sinne (z.B. Sarcoptes scabiei, Dermanyssus gallinae).

Die erfindungsgemäßen Wirkstoffe, die unverdünnt oder vorzugsweise verdünnt eingesetzt werden können, lassen sich in die für Repellents üblichen Formulierungen überführen. Sie lassen sich in allen in der Kosmetik üblichen Darreichungsformen einsetzen, beispielsweise in Form von Lösungen, Emulsionen, Gelen, Salben, Pasten, Cremes, Pulvern, Stiften, Sprays oder Aerosolen aus Sprühdosen.

Für die Anwendung im nichtkosmetischen Bereich lassen sich die Wirkstoffe z.B. in Granulate, Ölsprühmittel oder Slow-Release-Formulierungen einarbeiten.

Die Zubereitungen werden in bekannter Weise durch Vermischen oder Verdünnen der erfindungsgemäßen Wirkstoffe mit Lösungsmitteln (z.B. Xylol, Chlorbenzole, Paraffine, Methanol, Ethanol, Isopropanol, Wasser), Trägerstoffe (z.B. Kaoline, Tonerden, Talkum, Kreide, hochdisperse Kieselsäure, Silikate), Emulgiermitteln (z.B. Polyoxyethylen-Fettsäure-Ester, Polyoxyethylen-Fettalkohol-Ether, Alkylsulfonate, Arylsulfonate) und Dispergiermitteln (z.B. Lignin, Sulfitablaugen, Methylcellulose) hergestellt.

Die erfindungsgemäßen Wirkstoffe können in den Formulierungen miteinander gemischt oder auch in Mischungen mit anderen bekannten Wirkstoffen (z.B. Sonnenschutzmittel) eingesetzt werden. Die Zubereitungen enthalten im allgemeinen zwischen 0,1 und 95 Gew.-% Wirkstoff, vorzugsweise zwischen 0,5 und 90 %.

Zum Schutz gegen blutsaugende Insekten oder Milben werden die erfindungsgemäßen Wirkstoffe entweder auf die menschliche oder tierische Haut aufgebracht oder Kleidungsstücke und andere Gegenstände damit behandelt.

Auch als Zusatz zu Imprägniermitteln für beispielsweise Textilbahnen, Kleidungsstücke, Verpackungsmaterialien, sowie als Zusatz zu Polier-, Putz- und Fensterreinigungsmitteln sind die erfindungsgemäßen Wirkstoffe geeignet.

Die folgenden Beispiele für die Zubereitungen und die Verwendung der erfindungsgemäßen Wirkstoffe dienen der weiteren Erläuterung der Erfindung.

### Formulierungs-Beispiel 1

Ein Repellentmittel in Form einer Lotion zur Anwendung auf der Haut wird hergestellt durch Vermischen von 30 Teilen eines der erfindungsgemäßen Wirkstoffe, 1,5 Teilen Parfüm und 68,5 Teilen Isopropanol. Isopropanol kann durch Ethanol ersetzt werden.

### Formulierungs-Beispiel 2

Ein Repellentmittel in Form eines Aerosols zum Aufsprühen auf die Haut wird hergestellt, indem man 50 % Wirkstofflösung, bestehend aus 30 Teilen eines der erfindungsgemäßen Wirkstoffe, 1,5 Teilen Parfüm, 68,5 Teilen Isopropanol, mit 50 % Frigen 11/12 (= halogenierter Kohlenwasserstoff als Treibgas) als Sprühdose formuliert.

### Formulierungs-Beispiel 3

Eine andere Sprühdose setzt sich aus 40 % Wirkstofflösung, bestehend aus 20 Teilen eines der erfindungsgemäßen Wirkstoffe, 1 Teil Parfüm, 79 Teilen Isopropanol und 60 % Propan/Butan (Verhältnis 15:85) zusammen.

Es wurden individuelle Formulierungen entsprechend den Formulierungs-Beispielen 1, 2 und 3 unter Einsatz folgender Wirkstoffe hergestellt: Verbindungen gemäß den Beispielen 1, 7, 18 und 23.

### Beispiel A

### Repellenttest am Meerschweinchen

Testtier: Aedes aegypti (Imagines)
Zahl der Testtiere: ca. 5.000
Lösungsmittel: Ethanol (99,8 %)

3 Gewichtsteile Wirkstoff werden in 100 Volumenteilen Lösungsmittel aufgenommen.

Ein Meerschweinchen, dessen Rücken in einem Bereich von 50 cm² rasiert worden ist, wird in einem engen Käfig (Box) so fixiert, daß nur die rasierte Fläche den Mücken zugänglich ist. Nach Behandlung der Fläche mit 0,4 ml Wirkstofflösung wird das Meerschweinchen nach Verdunsten des Lösungsmittels samt Box in einen 60 x 60 x 60 cm messenden Käfig gestellt, der nur mit Zuckerwasser gefütterte Testtiere beiderlei Geschlechts enthält.

Es wird für 10 Minuten beobachtet, wieviele Mücken das Meerschweinchen stechen.

Anschließend wird dieses herausgenommen und der Test nach einer Stunde wiederholt. Der Versuch wird maximal 9 Stunden lang durchgeführt und so lange, bis die Wirkung abbricht.

Hierbei zeigen die Verbindungen gemäß folgender Beispiele eine Repellentwirkung, die besser ist als der Stand der Technik (DEET): Beispiele 1, 7, 18 und 23.

### Beispiel B

### Repellenttest am Meerschweinchen

Testtier: Culex pipiens fatigans (Imagines)
Zahl der Testtiere: ca. 1.000
Lösungsmittel: Ethanol (99,8 %)

1 Gewichtsteil Wirkstoff wird in 100 Volumenteilen Lösungsmittel aufgenommen.

Ein Meerschweinchen, dessen Rücken in einem Bereich von 50 cm² rasiert worden ist, wird in einem engen Käfig (Box) so fixiert, daß nur die rasierte Fläche den Mücken zugänglich ist. Nach Behandeln der Fläche mit 0,4 ml Wirkstofflösung wird das Meerschweinchen nach Verdunsten des Lösungsmittels samt Bos in einen 60 x 60 x 60 cm messenden Käfig gestellt, der nur mit Zuckerwasser gefütterte Testtiere beiderlei Geschlechts enthält.

Es wird für 10 Minuten beobachtet, wieviele Mücken das Meerschweinchen stechen.

Anschließend wird dieses herausgenommen und der Test nach einer Stunde wiederholt. Der Versuch wird maximal 9 Stunden lang durchgeführt oder so lange, bis die Wirkung abbricht.

Hierbei zeigen die Verbindungen gemäß folgender Beispiele eine Wirkung als Repellent, die besser ist als der Stand der Technik (DEET): Bsp. Nr, 1.

### Beispiel C

### Repellenttest am Meerschweinchen

Testtier: Stomoxys calcitrans (Imagines)
Zahl der Testtiere: ca. 1.000
Lösungsmittel: Ethanol (99,8 %)

5 Gew.-Teile Wirkstoff werden in 100 Volumenteilen Lösungsmittel aufgenommen.

Ein Meerschweinchen, dessen Rücken in einem Bereich von 50 cm² rasiert worden ist, wird in einem engen Käfig (Box) so fixiert, daß nur die rasierte Fläche den Mücken zugänglich ist. Nach Behandeln der Fläche mit 0,4 ml Wirkstofflösung wird das Meerschweinchen nach Verdunsten des Lösungsmittels samt Box in einen 60 x 60 x 60 cm messenden Käfig gestellt, der nur mit Zuckerwasser gefütterte Testtiere beiderlei Geschlechts enthält.

Es wird für 10 Minuten beobachtet, wieviele Mücken das Meerschweinchen stechen.

Anschließend wird dieses herausgenommen und der Test nach einer Stunde wiederholt. Der Versuch wird maximal 9 Stunden lang durchgeführt oder so lange, bin die Wirkung abbricht.

Hierbei zeigen die Verbindungen gemäß folgender Beispiele eine Wirkung als Repellent, die besser ist als der Stand der Technik (DEET): Bsp. Nr. 1.

### Herstellungsbeispiele

### Beispiel 1

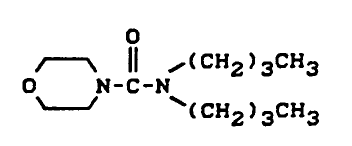

10,4 g (0,12 mol) Morpholin, 0,2 g 4-Dimethylamino-pyridin und 15,2 g (0,15 mol) Triethylamin werden in 250 ml absolutem Toluol gelöst. Bei 0°C tropft man 20 g (0,1 mol) N,N-Dibutylcarbamoylchlorid langsam zu. Man rührt 14 Stunden bei 20°C und anschließend 2 Stunden bei 30°C nach. Das ausgefallene Triethylammoniumchlorid wird abgesaugt. Das Filtrat wird mit verdünnter Salzsäure und Wasser gewaschen, über Natriumsulfat getrocknet und das Lösungsmittel im Vakuum abgedampft. Kugelrohrdestillation ergibt 21,8 g (86 % der Theorie) 4-(N,N-Dibutylamino-carbonyl)-morpholin als farbloses Öl mit dem Siedepunkt 140°C bei 0,2 Torr.

### Beispiel 2

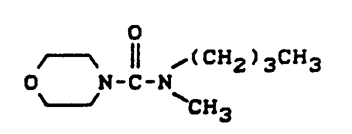

Unter Stickstoffatmosphäre werden 5 g (27 mmol) 4-(N-Butylaminocarbonyl)-morpholin in 15 ml absolutem Dimethylformamid gelöst. Bei 0°C gibt man vorsichtig 1,7 g (57 mmol) Natriumhydrid (80 %ig) zu. Man setzt 5,7 g (40 mmol) Methyljodid zu und rührt 2 Stunden bei 40°C.

Unter Kühlung werden 2 ml Wasser zugegeben und das Lösungsmittel im Vakuum abgedampft. Der Rückstand wird in Dichlormethan gelöst, mit Wasser gewaschen und nach Trocknen über Natriumsulfat das Lösungsmittel abgezogen. Kugelrohrdestillation ergibt 4,6 g (86 % der Theorie) 4-(N-Butyl-N-methylamino-carbonyl)-morpholin in Form eines Öls mit dem Siedepunkt 110°C bei 0,15 Torr.

### Beispiel 3

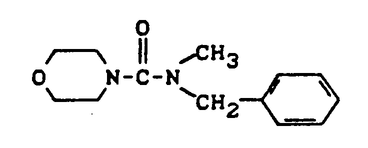

50 ml N-Methylbenzylamin werden bei 20°C langsam mit 3 g (0,026 Mol) 4-Chlorcarbonyl-morpholin versetzt (exotherme Reaktion). Man rührt 4 Stunden bei 40°C und filtriert dann direkt über Kieselgel (Laufmittel = Toluol : Aceton = 7:3). Nach Einrotieren des Lösungsmittels erhält man 4,4 g (77 % d.Th.) 4-(N-Benzyl-N-methyl-aminocarbonyl)-morpholin mit einem Brechungsindex n$\frac{\text{20}}{\text{D}}$ = 1,5430.

In analoger Weise zu den Beispielen 1, 2 und 3 konnten die folgenden Verbindungen erhalten werden.

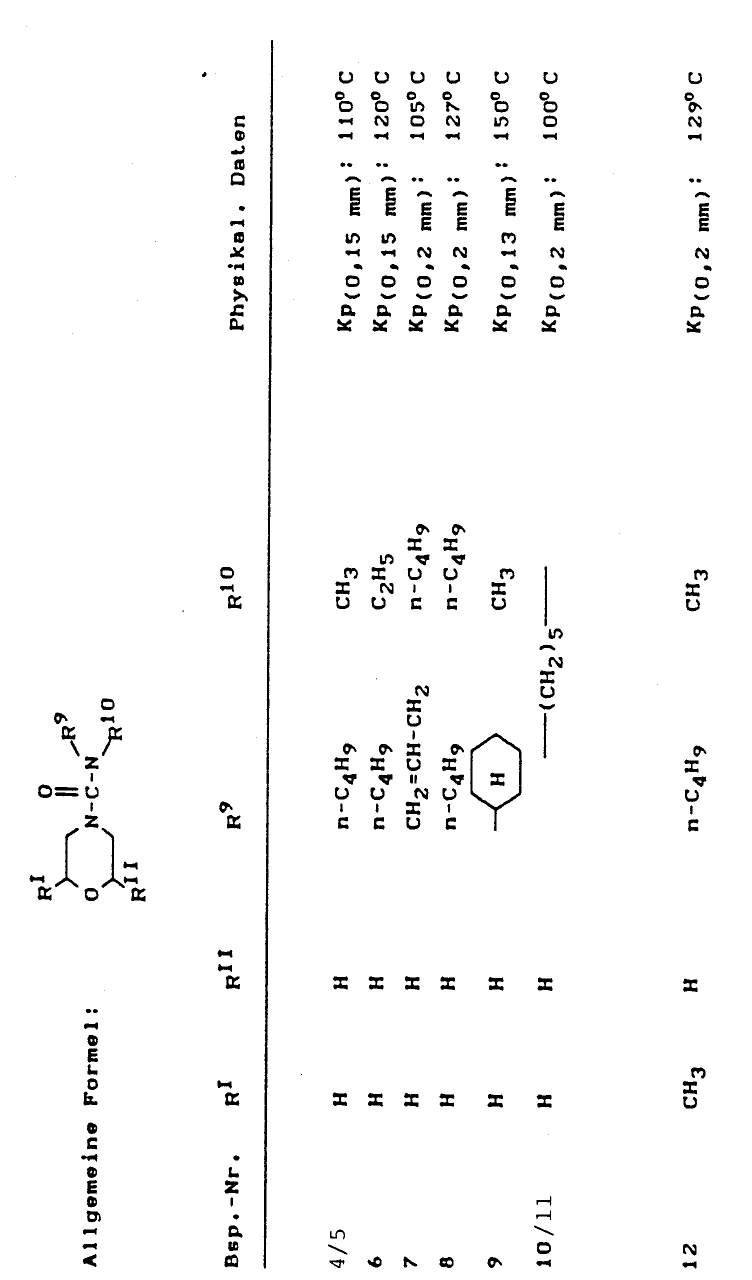

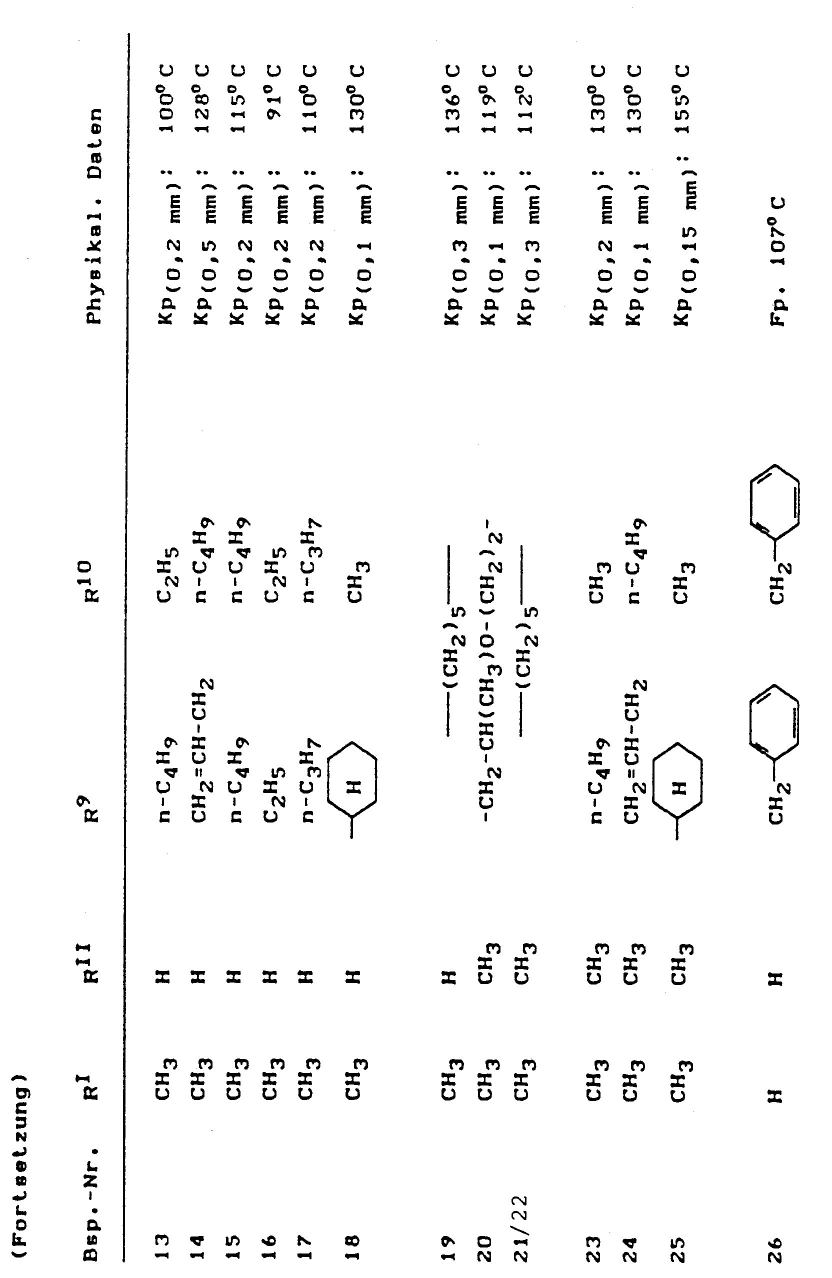

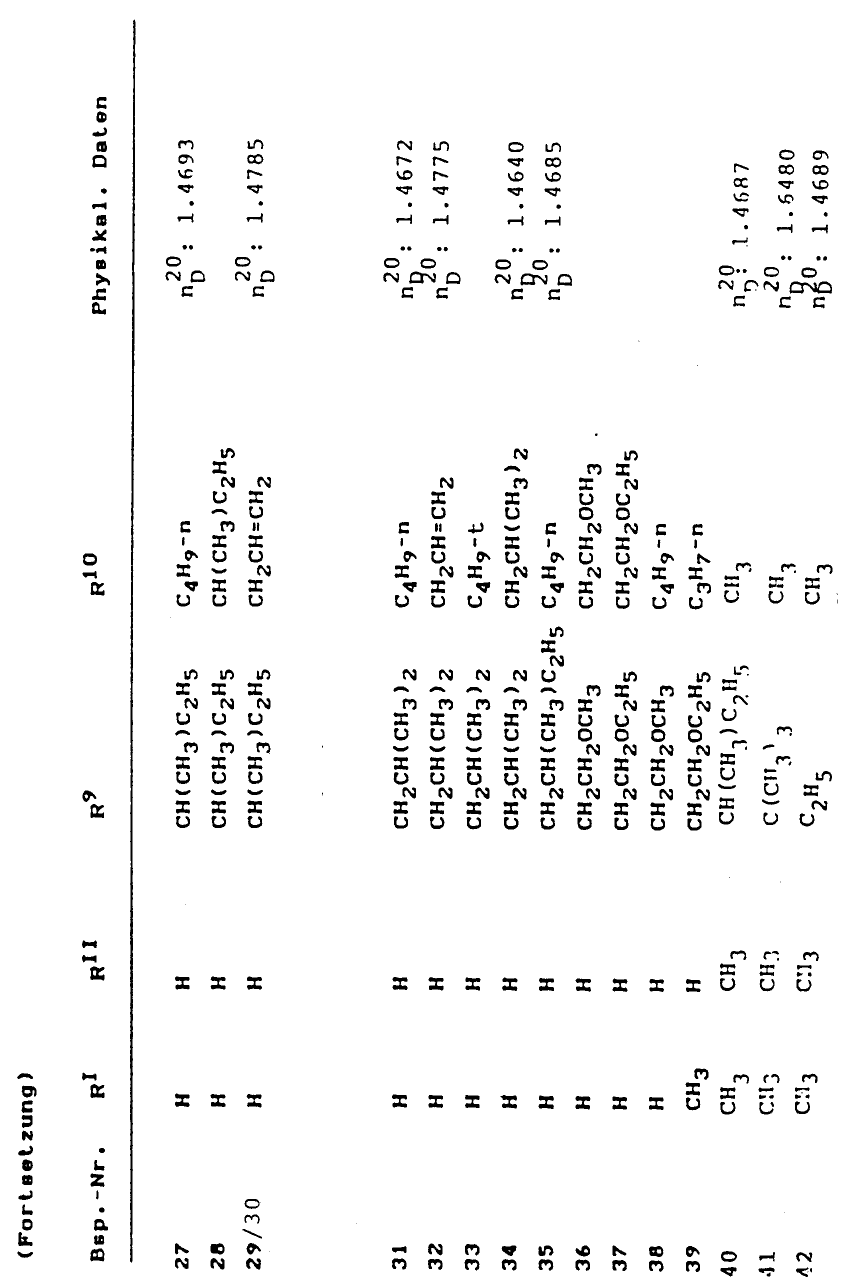

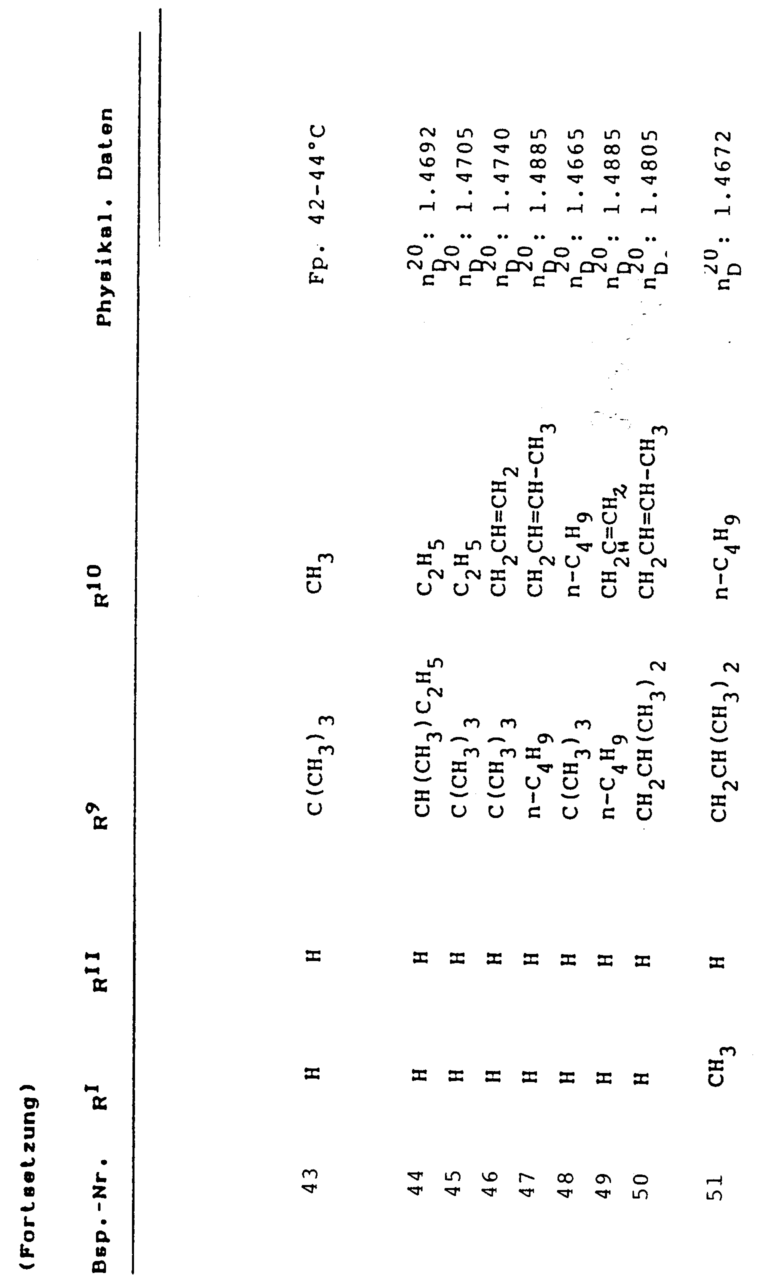

## Patentansprüche (Patentansprüche für folgende(n) Vertragsstaat(en): AT, BE, CH, LI, DE, DK, FR, GB, GR, IT, LU, NL, SE)

1. Morpholinoharnstoff-Derivate der Formel (I) 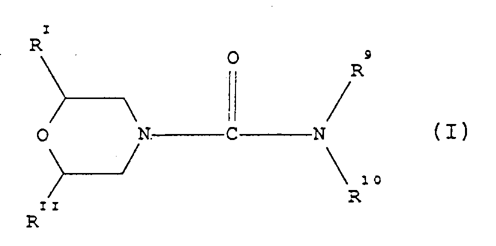 in welcher
R^{I} und R^{II} gleich oder verschieden sind und für Wasserstoff, C₁-C₆-Alkyl, C₂-C₆-Alkenyl, C₃-C₆-Cycloalkyl oder Phenyl stehen,
R⁹ für gegebenenfalls durch C₁-C₄-Alkoxy oder Halben substituiertes C₁-C₆-Alkyl, Alkenyl mit bis zu 6 C-Atomen, C₃-C₆-Cycloalkyl, Benzyl oder Cyclohexylmethyl steht,
R¹⁰ für gegebenenfalls durch C₁-C₄-Alkoxy oder Halogen substituiertes C₁-C₆-Alkyl oder R⁹ und R¹⁰ zusammen für einen gegebenenfalls durch Sauerstoff unterbrochenen und gegebenenfalls durch C₁-C₄-Alkyl substituierten Tetramethylen-, Pentamethylen-, Hexamethylen-oder Heptamethylenrest stehen,
wobei solche Verbindungen der Formel (I) ausgenommen sind, in welchen zugleich
a)
R^{I} und R^{II} für Wasserstoff und
R⁹ und R¹⁰ für Methyl oder Ethyl stehen und
b)
R^{I} und R^{II} für Wasserstoff stehen und
R⁹ und R¹⁰ einen Morpholinring bilden.

2. Morpholinoharnstoff-Derivate gemäß Anspruch 1, in welcher
R^{I} und R^{II} gleich oder verschieden sind und für Wasserstoff, Methyl oder Ethyl stehen,
R⁹ für C₁-C₆-Alkyl, Alkenyl mit bis zu 6 C-Atomen oder C₃-C₆-Cycloalkyl steht und
R¹⁰ für C₁-C₆-Alkyl steht.

3. Verfahren zur Herstellung der Morpholinoharnstoff-Derivate gemäß Anspruch 1
dadurch gekennzeichnet, daß man entweder
a) Morpholine der Formel (II) 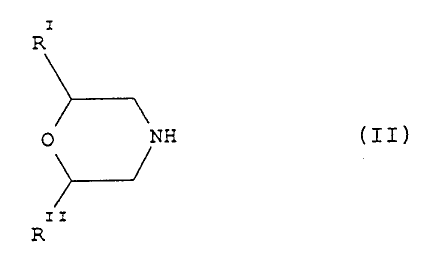 worin
R^{I} und R^{II} die oben angegebene Bedeutung haben,
mit Carbamidsäurederivaten der Formel (III) 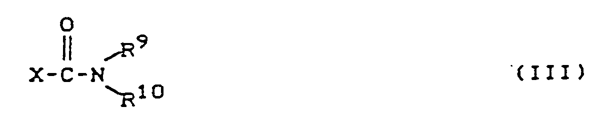 in welcher
R⁹ und R¹⁰ die oben angegebene Bedeutung besitzen und
X für eine Abgangsgruppe steht,
in Gegenwart einer Base und in Gegenwart eines Lösungsmittels umsetzt,
oder daß man
b) zunächst Morpholine der Formel (II) 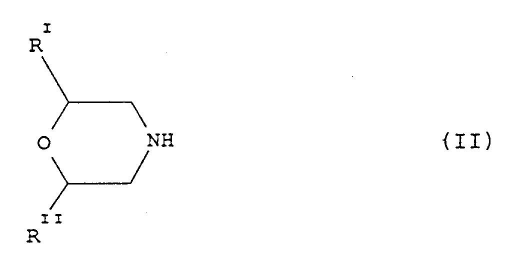 worin
R^{I} und R^{II} die oben angegebene Bedeutung haben,
mit Isocyanaten der Formel (IV)
O=C=N-R⁹ (IV)
worin
R⁹ die oben angegebene Bedeutung besitzt,
in einem Lösungsmittel umsetzt und das erhaltene Produkt der Formel (V) 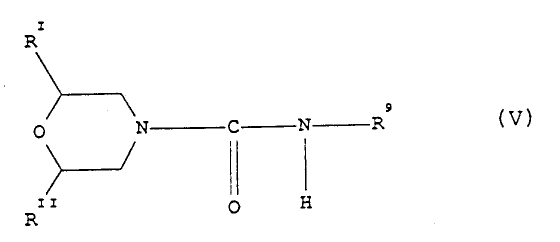 mit einem Alkylierungsmittel der Formel (VI)
R¹⁰-X (VI)
worin
R¹⁰ die oben angegebene Bedeutung besitzt und X für eine Abgangsgruppe steht,
in einem Lösungsmittel in Gegenwart einer Base umsetzt,
oder daß man
c) 4-Halogencarbonylmorpholin-Derivate der Formel (VII) 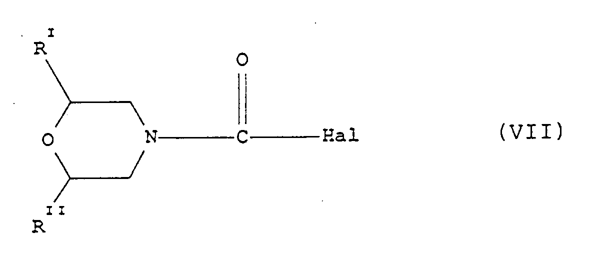 worin
R^{I} und R^{II} die obengenannte Bedeutung haben und Hal für Halogen steht,
mit sekundären Aminen der Formel (VIII) 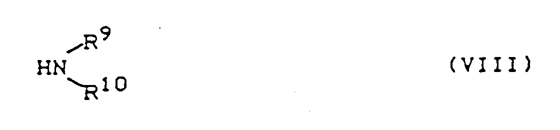 worin
R⁹ und R¹⁰ die obengenannte Bedeutung haben,
gegebenenfalls in Gegenwart von Basen und gegebenenfalls in Gegenwart von Lösungsmitteln umsetzt.

4. Insekten- und milbenabwehrende Mittel, gekennzeichnet durch einen Gehalt an mindestens einem Morpholinoharnstoff-Derivat gemäß Anspruch 1.

5. Verfahren zur Herstellung von insekten- und milbenabwehrenden Mitteln, dadurch gekennzeichnet, daß man Morpholinoharnstoff-Derivate gemäß Anspruch 1 mit Streckmitteln und/oder oberflächenaktiven Mitteln vermischt.

6. Nichttherapeutisches Verfahren zur Abwehr von Insekten und Milben, dadurch gekennzeichnet, daß man Morpholinoharnstoff-Derivate gemäß Anspruch 1, eingeschlossen die Verbindungen der Formel (I), in welchen zugleich
a)
R^{I} und R^{II} für Wasserstoff und
R⁹ und R¹⁰ für Methyl oder Ethyl stehen und
b)
R^{I} und R^{II} für Wasserstoff stehen und
R⁹ und R¹⁰ einen Morpholinring bilden,
auf Insekten und/oder Milben und/oder ihren Lebensraum einwirken läßt.

7. Nichttherapeutische Verwendung von Morpholinoharnstoff-Derivaten gemäß Anspruch 1, eingeschlossen die Verbindungen der Formel (I), in welchen zugleich
a)
R^{I} und R^{II} für Wasserstoff und
R⁹ und R¹⁰ für Methyl oder Ethyl stehen und
b)
R^{I} und R^{II} für Wasserstoff stehen und
R⁹ und R¹⁰ einen Morpholinring bilden,
zur Abwehr von Insekten und Milben.

8. Morpholinoharnstoff-Derivate gemäß Anspruch 1, eingeschlossen die Verbindungen der Formel (I), in welchen zugleich
a)
R^{I} und R^{II} für Wasserstoff und
R⁹ und R¹⁰ für Methyl stehen und
b)
R^{I} und R^{II} für Wasserstoff stehen und
R⁹ und R¹⁰ einen Morpholinring bilden,
zur Verwendung als Mittel zur Abwehr von Insekten und Milben.

9. Verwendung von Morpholinoharnstoff-Derivaten gemäß Anspruch 1, eingeschlossen die Verbindungen der Formel (I), in welchen zugleich
a)
R^{I} und R^{II} für Wasserstoff und
R⁹ und R¹⁰ für Methyl oder Ethyl stehen und
b)
R^{I} und R^{II} für Wasserstoff stehen und
R⁹ und R¹⁰ einen Morpholinring bilden,
zur Herstellung von Mitteln zur Abwehr von Insekten und Milben.

## Patentansprüche (Patentansprüche für folgende(n) Vertragsstaat(en): ES)

1. Verfahren zur Herstellung der Morpholinoharnstoff-Derivate der Formel (I) 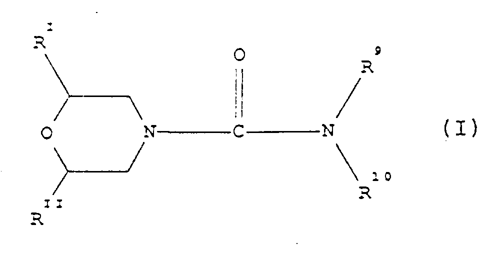 in welcher
R^{I} und R^{II} gleich oder verschieden sind und für Wasserstoff, C₁-C₆-Alkyl, C₂-C₆-Alkenyl, C₃-C₆-Cycloalkyl oder Phenyl stehen,
R⁹ gegebenenfalls durch C₁-C₄-Alkoxy oder Halogen substituiertes C₁-C₆-Alkyl, Alkenyl mit bis zu 6 C-Atomen, C₃-C₆-Cycloalkyl, Benzyl oder Cyclohexylmethyl steht,
R¹⁰ für gegebenenfalls durch C₁-C₄-Alkoxy oder Halogen-substituiertes C₁-C₆-Alkyl oder R⁹ und R¹⁰ zusammen für einen gegebenenfalls durch Sauerstoff unterbrochenen und gegebenenfalls durch C₁-C₄-Alkyl substituierten Tetramethylen-, Pentamethylen-, Hexamethylen- oder Heptamethylenrest stehen.
wobei solche Verbindungen der Formel (I) ausgenommen sind, in welchen zugleich
a)
R^{I} und R^{II} für Wasserstoff und
R⁹ und R¹⁰ für Methyl oder Ethyl stehen und
b)
R^{I} und R^{II} für Wasserstoff stehen und
R⁹ und R¹⁰ einen Morpholinring bilden,
dadurch gekennzeichnet, daß man entweder
a) Morpholine der Formel (II) 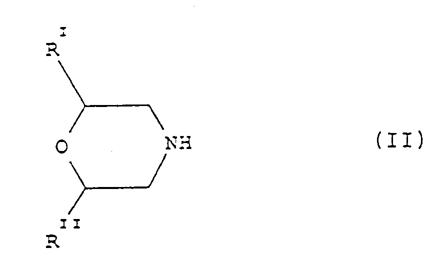 worin
R^{I} und R^{II} die oben angegebene Bedeutung haben,
mit Carbamidsäurederivaten der Formel (III) 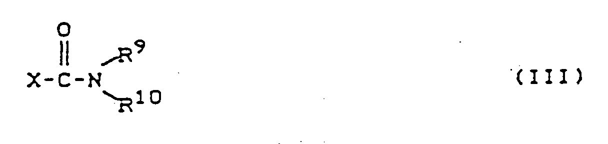 in welcher
R⁹ und R¹⁰ die oben angegebene Bedeutung besitzen und
X für eine Abgangsgruppe steht,
in Gegenwart einer Base und in Gegenwart eines Lösungsmittels umsetzt,
oder daß man
b) zunächst Morpholine der Formel (II) 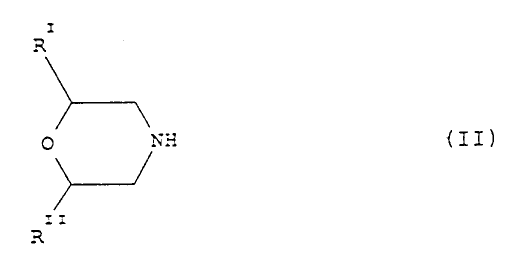 worin
R^{I} und R^{II} die oben angegebene Bedeutung haben,
mit Isocyanaten der Formel (IV)
O=C=N-R⁹ (IV)
worin
R⁹ die oben angegebene Bedeutung besitzt, in einem Lösungsmittel umsetzt und das erhaltene Produkt der Formel (V) 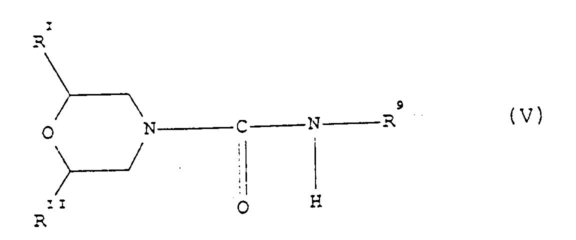 mit einem Alkylierungsmittel der Formel (VI)
R¹⁰-X (VI)
worin
R¹⁰ die oben angegebene Bedeutung besitzt und X für eine Abgangsgruppe steht,
in einem Lösungsmittel in Gegenwart einer Base umsetzt,
oder daß man
c) 4-Halogencarbonylmorpholin-Derivate der Formel (VII) 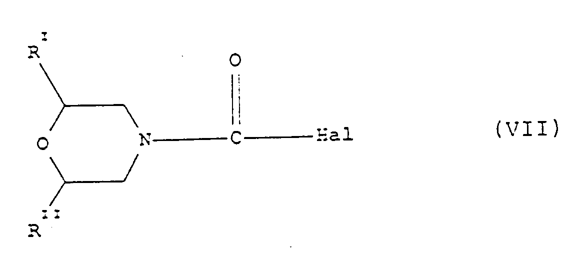 worin
R^{I} und R^{II} die obengenannte Bedeutung haben und Hal für Halogen steht,
mit sekundären Aminen der Formel (VIII) 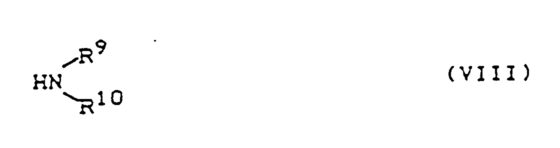 worin
R⁹ und R¹⁰ die obengenannte Bedeutung haben,
gegebenenfalls in Gegenwart von Basen und gegebenenfalls in Gegenwart von Lösungsmitteln umsetzt.

2. Verfahren gemäß Anspruch 1, wobei in Formel (I)
R^{I} und R^{II} gleich oder verschieden sind und für Wasserstoff, Methyl oder Ethyl stehen,
R⁹ für C₁-C₆-Alkyl, C₂-C₆-AIkenyl oder C₃-C₆-Cycloalkyl steht und
R¹⁰ für C₁-C₆-Alkyl steht.

3. Insekten- und milbenabwehrende Mittel, gekennzeichnet durch einen Gehalt an mindestens einem Morpholinoharnstoff-Derivat gemäß Anspruch 1.

4. Verfahren zur Herstellung von insekten- und milbenabwehrenden Mitteln, dadurch gekennzeichnet, daß man Morpholinoharnstoff-Derivate gemäß Anspruch 1 mit Streckmitteln und/oder oberflächenaktiven Mitteln vermischt.

5. Nichttherapeutisches Verfahren zur Abwehr von Insekten und Milben, dadurch gekennzeichnet, daß man Morpholinoharnstoff-Derivate gemäß Anspruch 1, eingeschlossen die Verbindungen der Formel (I), in welchen zugleich
a)
R^{I} und R^{II} für Wasserstoff und
R⁹ und R¹⁰ für Methyl oder Ethyl stehen und
b)
R^{I} und R^{II} für Wasserstoff stehen und
R⁹ und R¹⁰ einen Morpholinring bilden,
auf Insekten und/oder Milben und/oder ihren Lebensraum einwirken läßt.

6. Nichttherapeutische Verwendung von Morpholinoharnstoff-Derivaten gemäß Anspruch 1, eingeschlossen die Verbindungen der Formel (I), in welchen zugleich
a)
R^{I} und R^{II} für Wasserstoff und
R⁹ und R¹⁰ für Methyl oder Ethyl stehen und
b)
R^{I} und R^{II} für Wasserstoff stehen und
R⁹ und R¹⁰ einen Morpholinring bilden,
zur Abwehr von Insekten und Milben.

7. Morpholinoharnstoff-Derivate gemäß Anspruch 1, eingeschlossen die Verbindungen der Formel (I), in welchen zugleich
a)
R^{I} und R^{II} für Wasserstoff und
R⁹ und R¹⁰ für Methyl stehen und
b)
R^{I} und R^{II} für Wasserstoff stehen und
R⁹ und R¹⁰ einen Morpholinring bilden,
zur Verwendung als Mittel zur Abwehr von Insekten und Milben.

8. Verwendung von Morpholinoharnstoff-Derivaten gemäß Anspruch 1, eingeschlossen die Verbindungen der Formel (I), in welchen zugleich
a)
R^{I} und R^{II} für Wasserstoff und
R⁹ und R¹⁰ für Methyl oder Ethyl stehen und
b)
R^{I} und R^{II} für Wasserstoff stehen und
R⁹ und R¹⁰ einen Morpholinring bilden,
zur Herstellung von Mitteln zur Abwehr von Insekten und Milben.

## Claims (Claims for the following Contracting State(s): AT, BE, CH, LI, DE, DK, FR, GB, GR, IT, LU, NL, SE)

1. Morpholinourea derivatives of the formula (I) 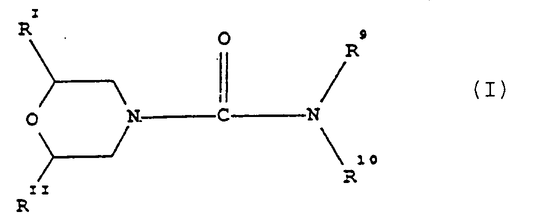 in which
R^{I} and R^{II} are identical or different and represent hydrogen, C₁-C₆-alkyl, C₂-C₆-alkenyl, C₃-C₆-cycloalkyl or phenyl and
R⁹ represents optionally C₁-C₄-alkoxy-substituted or halogen-substituted C₁-C₆-alkyl, alkenyl having up to 6 C atoms, C₃-C₆-cycloalkyl, benzyl or cyclohexylmethyl,
R¹⁰ represents optionally C₁-C₄-alkoxy-substituted or halogen-substituted C₁-C₆-alkyl,
or R⁹ and R¹⁰ together represent a tetramethylene, pentamethylene, hexamethylene or heptamethylene radical which is optionally interrupted by oxygen and optionally substituted by C₁-C₄-alkyl, with the exception of those compounds of the formula (I) in which simultaneously
a)
R^{I} and R^{II} represent hydrogen and
R⁹ and R¹⁰ represent methyl or ethyl and
b)
R^{I} and R^{II} represent hydrogen and
R⁹ and R¹⁰ form a morpholine ring.

2. Morpholinourea derivatives according to Claim 1 in which
R^{I} and R^{II} are identical or different and represent hydrogen, methyl or ethyl,
R⁹ represents C₁-C₆-alkyl, alkenyl having up to 6 C atoms or C₃-C₆-cycloalkyl and
R¹⁰ represents C₁-C₆-alkyl.

3. Process for the preparation of the morpholinourea derivatives according to Claim 1
characterised in that either
a) morpholines of the formula (II) 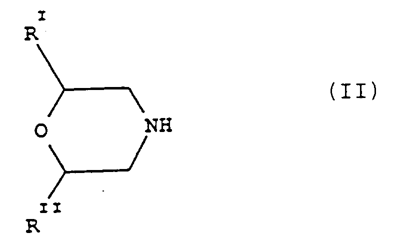 where
R^{I} and R^{II} have the abovementioned meaning,
are reacted with carbamic acid derivatives of the formula (III) 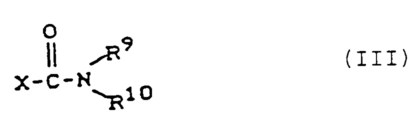 in which
R⁹ and R¹⁰ have the abovementioned meaning and
X represents a leaving group,
in the presence of a base and in the presence of a solvent,
or in that
b) morpholines of the formula (II) 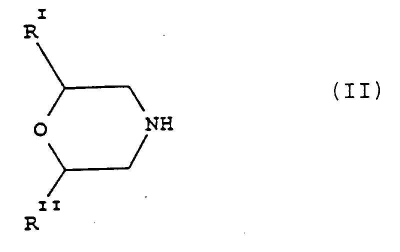 where
R^{I} and R^{II} have the abovementioned meaning,
are first reacted with isocyanates of the formula (IV)
O=C=N-R⁹ (IV)
where
R⁹ has the abovementioned meaning,
in a solvent, and the resulting product of the formula (V) 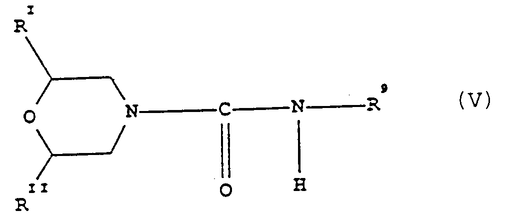 is reacted with an alkylating agent of the formula (VI)
R¹⁰-X (VI)
where
R¹⁰ has the abovementioned meaning and
X represents a leaving group,
in a solvent and in the presence of a base,
or in that
c) 4-halogenocarbonylmorpholine derivatives of the formula (VII) 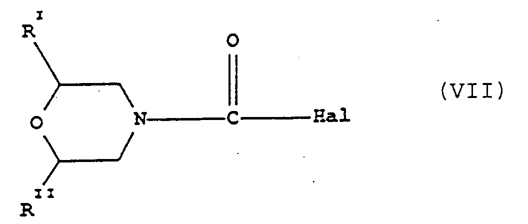 where
R^{I} and R^{II} have the abovementioned meaning, and Hal represents halogen,
are reacted with secondary amines of the formula (VIII) 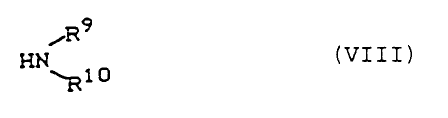 where
R⁹ and R¹⁰ have the abovementioned meaning,
if appropriate in the presence of bases and if appropriate in the presence of solvents.

4. Insect- and mite-repelling agents, characterised in that they contain at least one morpholinourea derivative according to Claim 1.

5. Process for the preparation of insect- and mite-repelling agents, characterised in that morpholinourea derivatives according to Claim 1 are mixed with extenders and/or surface-active agents.

6. Non-therapeutic process for repelling insects and mites, characterised in that morpholinourea derivatives according to Claim 1, including the compounds of the formula (I) in which simultaneously
a)
R^{I} and R^{II} represent hydrogen and
R⁹ and R¹⁰ represent methyl or ethyl and
b)
R^{I} and R^{II} represent hydrogen and
R⁹ and R¹⁰ form a morpholine ring,
are allowed to act on insects and/or mites and/or their environment.

7. Non-therapeutic use of morpholinourea derivatives according to Claim 1, including the compounds of the formula (I) in which simultaneously
a)
R^{I} and R^{II} represent hydrogen and
R⁹ and R¹⁰ represent methyl or ethyl and
b)
R^{I} and R^{II} represent hydrogen and
R⁹ and R¹⁰ form a morpholine ring,
for repelling insects and mites.

8. Morpholinourea derivatives according to Claim 1, including the compounds of the formula (I) in which simultaneously
a)
R^{I} and R^{II} represent hydrogen and
R⁹ and R¹⁰ represent methyl and
b)
R^{I} and R^{II} represent hydrogen and
R⁹ and R¹⁰ form a morpholine ring,
for use as insect and mite repellants.

9. Use of morpholinourea derivatives according to Claim 1, including the compounds of the formula (I) in which simultaneously
a)
R^{I} and R^{II} represent hydrogen and
R⁹ and R¹⁰ represent methyl or ethyl and
b)
R^{I} and R^{II} represent hydrogen and
R⁹ and R¹⁰ form a morpholine ring,
for the preparation of insect and mite repellants.

## Claims (Claims for the following Contracting State(s): ES)

1. Process for the preparation of the morpholinourea derivatives of the formula (I) 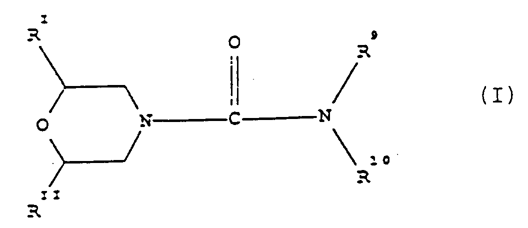 in which
R^{I} and R^{II} are identical or different and represent hydrogen, C₁-C₆-alkyl, C₂-C₆-alkenyl, C₃-C₆-cycloalkyl or phenyl and
R⁹ represents optionally C₁-C₄-alkoxy-substituted or halogen-substituted C₁-C₆-alkyl, alkenyl having up to 6 C atoms, C₃-C₆-cycloalkyl, benzyl or cyclohexylmethyl,
R¹⁰ represents optionally C₁-C₄-alkoxy-substituted or halogen-substituted C₁-C₆-alkyl,
or R⁹ and R¹⁰ together represent a tetramethylene, pentamethylene, hexamethylene or heptamethylene radical which is optionally interrupted by oxygen and optionally substituted by C₁-C₄-alkyl,
with the exception of those compounds of the formula (I) in which simultaneously
a)
R^{I} and R^{II} represent hydrogen and
R⁹ and R¹⁰ represent methyl or ethyl and
b)
R^{I} and R^{II} represent hydrogen and
R⁹ and R¹⁰ form a morpholine ring,
characterised in that either
a) morpholines of the formula (II) 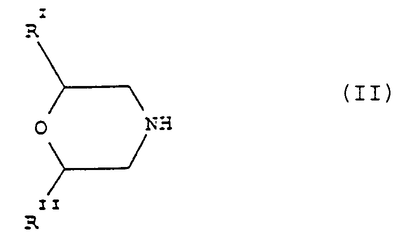 where
R^{I} and R^{II} have the abovementioned meaning,
are reacted with carbamic acid derivatives of the formula (III) 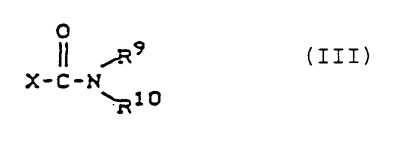 in which
R⁹ and R¹⁰ have the abovementioned meaning and
X represents a leaving group,
in the presence of a base and in the presence of a solvent,
or in that
b) morpholines of the formula (II) 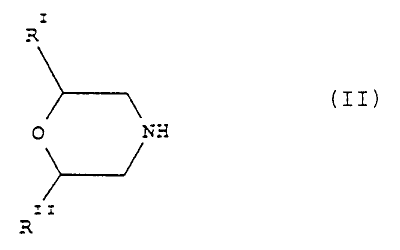 where
R^{I} and R^{II} have the abovementioned meaning,
are first reacted with isocyanates of the formula (IV)
O=C=N-R⁹ (IV)
where
R⁹ has the abovementioned meaning,
in a solvent, and the resulting product of the formula (V) 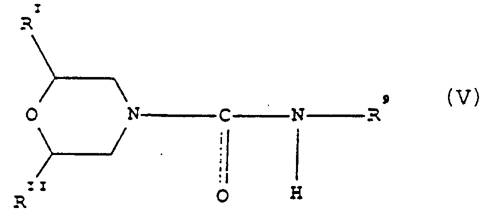 is reacted with an alkylating agent of the formula (VI)
R¹⁰-X (VI)
where
R¹⁰ has the abovementioned meaning and
X represents a leaving group, in a solvent and in the presence of a base,
or in that
c) 4-halogenocarbonylmorpholine derivatives of the formula (VII) 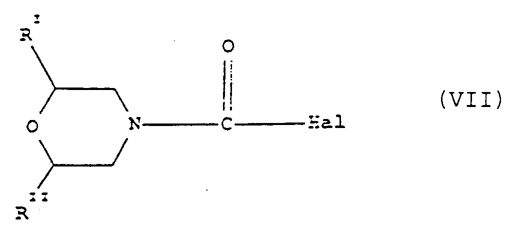 where
R^{I} and R^{II} have the abovementioned meaning, and Hal represents halogen,
are reacted with secondary amines of the formula (VIII) 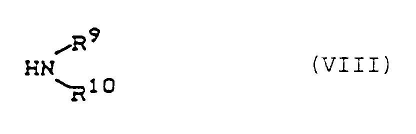 where
R⁹ and R¹⁰ have the abovementioned meaning,
if appropriate in the presence of bases and if appropriate in the presence of solvents.

2. Process according to Claim 1, with, in formula (I)
R^{I} and R^{II} being identical or different and representing hydrogen, methyl or ethyl,
R⁹ representing C₁-C₆-alkyl, C₂-C₆-alkenyl or C₃-C₆-cycloalkyl and
R¹⁰ representing C₁-C₆-alkyl.

3. Insect- and mite-repelling agents, characterised in that they contain at least one morpholinourea derivative according to Claim 1.

4. Process for the preparation of insect- and mite-repelling agents, characterised in that morpholinourea derivatives according to Claim 1 are mixed with extenders and/or surface-active agents.

5. Non-therapeutic process for repelling insects and mites, characterised in that morpholinourea derivatives according to Claim 1, including the compounds of the formula (I) in which simultaneously
a)
R^{I} and R^{II} represent hydrogen and
R⁹ and R¹⁰ represent methyl or ethyl and
b)
R^{I} and R^{II} represent hydrogen and
R⁹ and R¹⁰ form a morpholine ring,
are allowed to act on insects and/or mites and/or their environment.

6. Non-therapeutic use of morpholinourea derivatives according to Claim 1, including the compounds of the formula (I) in which simultaneously
a)
R^{I} and R^{II} represent hydrogen and
R⁹ and R¹⁰ represent methyl or ethyl and
b)
R^{I} and R^{II} represent hydrogen and
R⁹ and R¹⁰ form a morpholine ring,
for repelling insects and mites.

7. Morpholinourea derivatives according to Claim 1, including the compounds of the formula (I) in which simultaneously
a)
R^{I} and R^{II} represent hydrogen and
R⁹ and R¹⁰ represent methyl and
b)
R^{I} and R^{II} represent hydrogen and
R⁹ and R¹⁰ form a morpholine ring,
for use as insect and mite repellants.

8. Use of morpholinourea derivatives according to Claim 1, including the compounds of the formula (I) in which simultaneously
a)
R^{I} and R^{II} represent hydrogen and
R⁹ and R¹⁰ represent methyl or ethyl and
b)
R^{I} and R^{II} represent hydrogen and
R⁹ and R¹⁰ form a morpholine ring,
for the preparation of insect and mite repellants.

## Revendications (Revendications pour l'(les) Etat(s) contractant(s) suivant(s): AT, BE, CH, LI, DE, DK, FR, GB, GR, IT, LU, NL, SE)

1. Dérivés de morpholino-urée de formule (I) 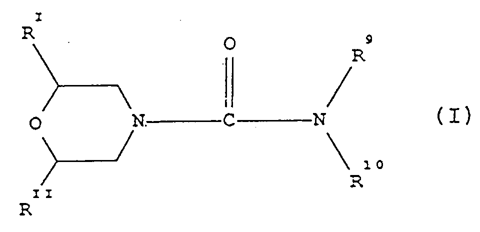 dans laquelle
R^{I} et R^{II} sont identiques ou différents et représentent de l'hydrogène, un reste alkyle en C₁ à C₆, alcényle en C₂ à C₆, cycloalkyle en C₃ à C₆ ou phényle,
R⁹ représente un reste, éventuellement substitué par un radical alkoxy en C₁ à C₄ ou par un halogène, alkyle en C₁ à C₆, alcényle ayant jusqu'à 6 atomes de carbone, cycloalkyle en C₃ à C₆, un reste benzyle ou un reste cyclohexylméthyle,
R¹⁰ représente un reste alkyle en C₁ à C₆ éventuellement substitué par un radical alkoxy en C₁ à C₄ ou par un halogène, ou bien
R⁹ et R¹⁰ forment ensemble un reste tétraméthylène, pentaméthylène, hexaméthylène ou heptaméthylène éventuellement interrompu par de l'oxygène et substitué le cas échéant par un radical alkyle en C₁ à C₄,
en excluant les composés de formule (I) dans lesquels, simultanément
a)
R^{I} et R^{II} représentent de l'hydrogène et
R⁹ et R¹⁰ représentent un groupe méthyle ou éthyle, et
b)
R^{I} et R^{II} représentent de l'hydrogène et
R⁹ et R¹⁰ forment un noyau de morpholine.

2. Dérivés de morpholino-urée suivant la revendication 1,
dans lesquels
R^{I} et R^{II} sont identiques ou différents et représentent de l'hydrogène, un groupe méthyle ou éthyle,
R⁹ est un groupe alkyle en C₁ à C₆, alcényle ayant jusqu'à 6 atomes de carbone ou cycloalkyle en C₃ à C₆ et
R¹⁰ est un groupe alkyle en C₁ à C₆.

3. Procédé de production des dérivés de morpholino-urée suivant la revendication 1,
caractérisé en ce que
a) on fait réagir des morpholines de formule (II) 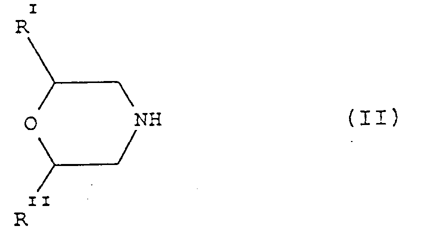 dans laquelle
R^{I} et R^{II} ont la définition indiquée ci-dessus, avec des dérivés d'acide carbamique de formule (III) 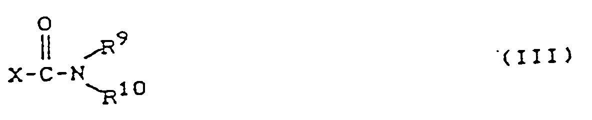 dans laquelle
R⁹ et R¹⁰ ont la définition indiquée ci-dessus et
X représente un groupe partant,
en présence d'une base et en présence d'un solvant,
ou bien
b) on fait réagir tout d'abord, dans un solvant, des morpholines de formule (II) 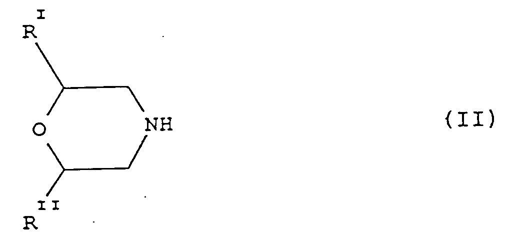 dans laquelle
R^{I} et R^{II} ont la définition indiquée ci-dessus, avec des isocyanates de formule (IV)
O=C=N-R⁹ (IV)
dans laquelle
R⁹ a la définition indiquée ci-dessus,
et on fait réagir dans un solvant, en présence d'une base, le produit obtenu de formule (V) 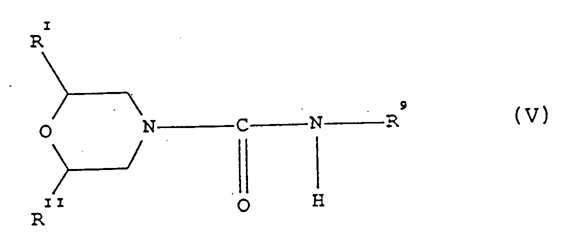 avec un agent alkylant de formule (VI)
R¹⁰-X (VI)
dans laquelle
R¹⁰ a la définition indiquée ci-dessus et X représente un groupe partant,
ou bien
c) on fait réagir des dérivés de 4-halogénocarbonylmorpholine de formule( VII) 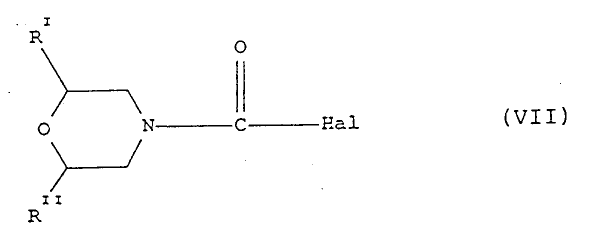 dans laquelle
R^{I} et R^{II} ont la définition indiquée ci-dessus et
Hal représente un halogène,
avec des amines secondaires de formule (VIII) 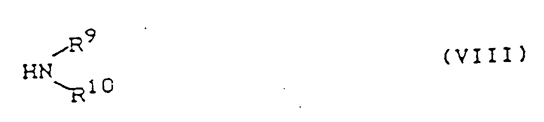 dans laquelle
R⁹ et R¹⁰ ont la définition indiquée ci-dessus, le cas échéant en présence de bases et en la présence éventuelle de solvants.

4. Composition à effet répulsif contre des insectes et des acariens, caractérisée par une teneur en au moins un dérivé de morpholino-urée suivant la revendication 1.

5. Procédé de préparation de compositions à effet répulsif contre des insectes et des acariens, caractérisé en ce qu'on mélange des dérivés de morpholino-urée suivant la revendication 1 avec des diluants et/ou des agents tensioactifs.

6. Procédé non thérapeutique de répulsion d'insectes et d'acariens, caractérisé en ce qu'on fait agir sur des insectes et/ou des acariens et/ou sur leur milieu, des dérivés de morpholino-urée suivant la revendication 1, y compris les composés de formule (I) dans lesquels, simultanément
a)
R^{I} et R^{II} représentent de l'hydrogène et
R⁹ et R¹⁰ représentent un groupe méthyle ou éthyle et
b)
R^{I} et R^{II} représentent de l'hydrogène et
R⁹ et R¹⁰ forment un noyau de morpholine,

7. Utilisation non thérapeutique de dérivés de morpholino-urée suivant la revendication 1, y compris les composés de formule (I) dans lesquels, simultanément
a)
R^{I} et R^{II} représentent de l'hydrogène et
R⁹ et R¹⁰ représentent un groupe méthyle ou éthyle et
b)
R^{I} et R^{II} représentent de l'hydrogène et
R⁹ et R¹⁰ forment un noyau de morpholine, pour la répulsion d'insectes et d'acariens.

8. Dérivés de morpholino-urée suivant la revendication 1, y compris les composés de formule (I), dans lesquels, simultanément
a)
R^{I} et R^{II} représentent de l'hydrogène et
R⁹ et R¹⁰ représentent un groupe méthyle et
b)
R^{I} et R^{II} représentent de l'hydrogène et
R⁹ et R¹⁰ forment un noyau de morpholine,
destinés à être utilisés comme répulsifs contre des insectes et des acariens.

9. Utilisation de dérivés de morpholino-urée suivant la revendication 1, y compris les composés de formule (I) dans lesquels, simultanément
a)
R^{I} et R^{II} représentent de l'hydrogène et
R⁹ et R¹⁰ représentent un groupe méthyle ou éthyle et
b)
R^{I} et R^{II} représentent de l'hydrogène et
R⁹ et R¹⁰ forment un noyau de morpholine,
pour la préparation de compositions destinées à produire un effet répulsif sur des insectes et des acariens.

## Revendications (Revendications pour l'(les) Etat(s) contractant(s) suivant(s): ES)

1. Procédé de production de dérivés de morpholino-urée de formule (I) 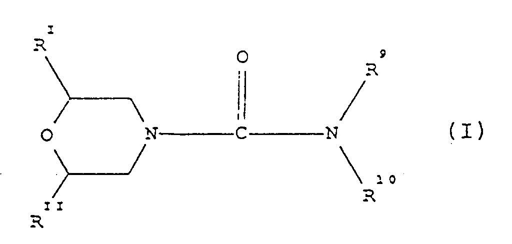 dans laquelle
R^{I} et R^{II} sont identiques ou différents et représentent de l'hydrogène, un reste alkyle en C₁ à C₆, alcényle en C₂ à C₆, cycloalkyle en C₃ à C₆ ou phényle,
R⁹ représente un reste, éventuellement substitué par un radical alkoxy en C₁ à C₄ ou par un halogène, alkyle en C₁ à C₆, alcényle ayant jusqu'à 6 atomes de carbone, cycloalkyle en C₃ à C₆, un reste benzyle ou un reste cyclohexylméthyle,
R¹⁰ représente un reste alkyle en C₁ à C₆ éventuellement substitué par un radical alkoxy en C₁ à C₄ ou par un halogène, ou bien
R⁹ et R¹⁰ forment ensemble un reste tétraméthylène, pentaméthylène, hexaméthylène ou heptaméthylène éventuellement interrompu par de l'oxygène et substitué le cas échéant par un radical alkyle en C₁ à C₄,
en excluant les composés de formule (I) dans lesquels, simultanément
a)
R^{I} et R^{II} représentent de l'hydrogène et
R⁹ et R¹⁰ représentent un groupe méthyle ou éthyle, et
b)
R^{I} et R^{II} représentent de l'hydrogène et
R⁹ et R¹⁰ forment un noyau de morpholine,
caractérisé en ce que
a) on fait réagir des morpholines de formule (II) 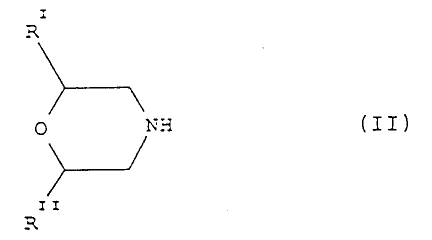 dans laquelle
R^{I} et R^{II} ont la définition indiquée ci-dessus, avec des dérivés d'acide carbamique de formule (III) 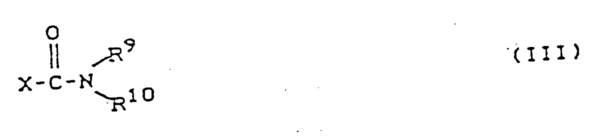 dans laquelle
R⁹ et R¹⁰ ont la définition indiquée ci-dessus et
X représente un groupe partant,
en présence d'une base et en présence d'un solvant,
ou bien
b) on fait réagir tout d'abord, dans un solvant, des morpholines de formule (II) 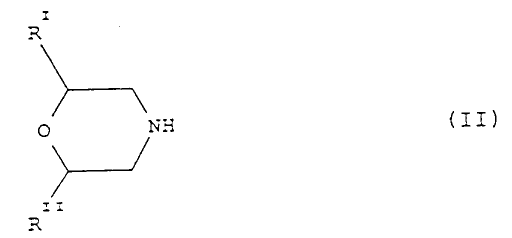 dans laquelle
R^{I} et R^{II} ont la définition indiquée ci-dessus, avec des isocyanates de formule (IV)
O=C=N-R⁹ (IV)
dans laquelle
R⁹ a la définition indiquée ci-dessus,
et on fait réagir dans un solvant en Présence d'une base le produit obtenu de formule (V) 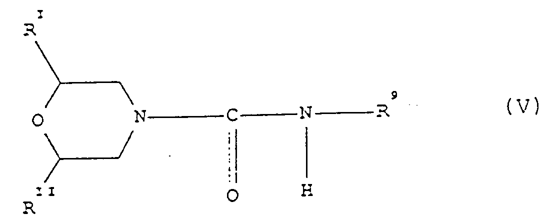 avec un agent alkylant de formule (VI)
R¹⁰-X (VI)
dans laquelle
R¹⁰ a la définition indiquée ci-dessus et
X représente un groupe partant,
ou bien
c) on fait réagir des dérivés de 4-halogénocarbonylmorphoiine de formule (VII) 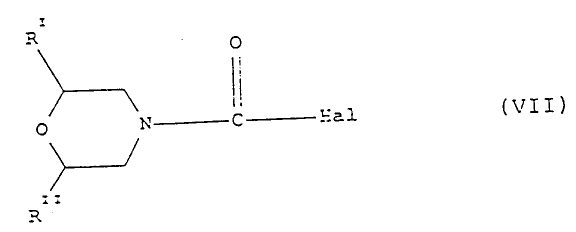 dans laquelle
R^{I} et R^{II} ont la définition indiquée ci-dessus, et Hal représente un halogène,
avec des amines secondaires de formule (VIII) 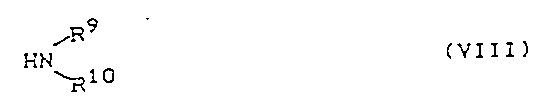 dans laquelle
R⁹ et R¹⁰ ont la définition indiquée ci-dessus, le cas échéant en présence de bases et en la présence éventuelle de solvants.

2. Procédé suivant la revendication 1, dans lequel, dans la formule (I),
R^{I} et R^{II} sont identiques ou différents et représentent de l'hydrogène, un groupe méthyle ou éthyle,
R⁹ est un groupe alkyle en C₁ à C₆, alcényle en C₂ à C₆ ou cycloalkyle en C₃ à C₆ et
R¹⁰ est un groupe alkyle en C₁ à C₆.

3. Compositions à effet répulsif contre des insectes et des acariens, caractérisées par une teneur en au moins un dérivé de morpholino-urée suivant la revendication 1.

4. Procédé de préparation de compositions à effet répulsif contre des insectes et des acariens, caractérisé en ce qu'on mélange des dérivés de morpholino-urée suivant la revendication 1 avec des diluants et/ou des agents tensio-actifs.

5. Procédé non thérapeutique de répulsion d'insectes et d'acariens, caractérisé en ce qu'on fait agir sur des insectes et/ou des acariens et/ou sur leur milieu des dérivés de morpholino-urée suivant la revendication 1, y compris les composés de formule (I) dans lesquels, simultanément
a)
R^{I} et R^{II} représentent de l'hydrogène et
R⁹ et R¹⁰ représentent un groupe méthyle ou éthyle et
b)
R^{I} et R^{II} représentent de l'hydrogène et
R⁹ et R¹⁰ forment un noyau de morpholine.

6. Utilisation non thérapeutique de dérivés de morpholino-urée suivant la revendication 1, y compris les composés de formule (I) dans lesquels, simultanément
a)
R^{I} et R^{II} représentent de l'hydrogène et
R⁹ et R¹⁰ représentent un groupe méthyle ou éthyle et
b)
R^{I} et R^{II} représentent de l'hydrogène, et
R⁹ et R¹⁰ forment un noyau de morpholine,
pour produire un effet répulsif sur des insectes et des acariens.

7. Dérivés de morpholino-urée suivant la revendication 1, y compris les composés de formule (I) dans lesquels, simultanément
a)
R^{I} et R^{II} représentent de l'hydrogène et
R⁹ et R¹⁰ représentent un groupe méthyle et
b)
R^{I} et R^{II} représentent de l'hydrogène, et
R⁹ et R¹⁰ forment un noyau de morpholine,
destinés à être utilisés comme agents répulsifs contre des insectes et des acariens.

8. Utilisation de dérivés de morpholino-urée suivant la revendication 1, y compris les composés de formule (I) dans lesquels, simultanément
a)
R^{I} et R^{II} représentent de l'hydrogène et
R⁹ et R¹⁰ représentent un groupe méthyle ou éthyle et
b)
R^{I} et R^{II} représentent de l'hydrogène, et
R⁹ et R¹⁰ forment un noyau de morpholine,
pour la préparation de compositions destinées à produire un effet répulsif sur des insectes et des acariens.
